# EUROPEAN PATENT APPLICATION

(11) **EP 2 983 128 A1**
(43) Date of publication of application: **10.02.2016**
(21) Application number: 13881319.1
(22) Date of filing: 01.04.2013
(51) Int. Cl.: G06Q 50/24, G06Q 50/22

(54) **MEDICAL-INFORMATION MANAGEMENT DEVICE, MEDICAL-INFORMATION MANAGEMENT SYSTEM, AND MEDICAL-INFORMATION MANAGEMENT METHOD**

(71) Applicant: Terumo Kabushiki Kaisha, Tokyo 151-0072 (JP)
(72) Inventor: TOUNOOKA Yuuya, Ashigarakami-gun Kanagawa 259-0151 (JP); YAMADA Kazuhiro, Ashigarakami-gun Kanagawa 259-0151 (JP)
(74) Representative: Casalonga
(86) International application number: PCT/JP2013/059995
(87) International publication number: WO 2014/162484

(57) **Abstract**

To provide a medical information management device and the like which are capable of facilitating detailed analysis and the like of drug administration information.

A medical information management device (10) stores, in association with individual drug information of each drug, degree-of-effect range information obtained by dividing a degree of effect of each drug into a plurality of pieces of degree-of-effect range information, and generates pieces of administration history information (41a) and (43a) of a drug A used at a medical instrument (70a) for each of the pieces of degree of effect information.

## Description

### Technical Field

The present invention relates to a medical information management device, a medical information management system, and a medical information management method for managing medical information such as drug information used in a hospital or the like, for example.

### Background Art

Conventionally, an infusion pump or the like is used at a hospital or the like to appropriately administer a drug to a patient (for example, Patent Literature 1).

Regarding drug information or the like that is used by a medical instrument such as the infusion pump, a database such as a "drug library" for managing the information is configured, and a system enabling appropriate administration of a drug or the like by the infusion pump or the like is adopted.

Also, drugs to be administered to patients and the like by such infusion pumps include those having efficacies and effects that are highly risky that a small dose may be fatal to a human.

Accordingly, administration history information has to be collected for such a drug, and administration information of the drug has to be analyzed on a regular basis.

Specifically, the administration information of a drug or the like is collected and analyzed for each operating room, an ICU (Intensive Care Unit) or the like, which is where the drug is to be used.

### Citation List

### Patent Literature

Patent Literature 1: JP 2011-87678 A

### Summary of Invention

### Technical Problem

However, depending on the drug, analysis or the like of more detailed administration information is necessary, but there is a problem that realization of such analysis is difficult.

Accordingly, the present invention has its object to provide a medical information management device, a medical information management system, and a medical information management method which are capable of facilitating detailed analysis and the like of administration information of a drug.

### Solution to Problem

In the present invention, the object mentioned above is achieved by a medical information management device, where the medical information management device stores, in association with individual drug information of each drug, degree-of-effect range information obtained by dividing a degree of effect of each drug into a plurality of pieces of degree-of-effect range information, and where the medicalinformation management device generates administration history information of the drug used at a medical instrument for each of the pieces of degree of effect information.

According to the configuration described above, the administration history information of a drug used at the medical instrument is generated for each piece of degree of effect information.

Accordingly, analysis and the like of more detailed administration information of each drug having specific degree of effect information are enabled.

The degree-of-effect range information is preferably color information, and the administration history information preferably includes warning information generated at a time of use of the drug at the medical instrument, and administration record information of the drug, and the warning information and the administration record information are preferably displayed on a display unit together with the color information.

According to the configuration described above, the degree-of-effect range information is color information, and the administration history information includes warning information generated at a time of use of the drug at the medical instrument, and administration record information of the drug.

Accordingly, the warning information and the administration record information, at the medical instrument, of each drug having specific degree of effect information may be obtained.

Also, these pieces of warning information and administration record information are displayed on a display unit together with the color information, and are thus easily viewed and easily understood by a user.

The warning information is preferably generated in association with cause information regarding the warning information.

According to the configuration described above, the warning information is generated in association with cause information regarding the warning information, and a user is enabled to view and understand the cause information regarding the warning information at the same time.

Preferably, there are provided a third medical information storage unit for storing user identification information for identifying a user who uses medical information, a plurality of pieces of first medical information obtained by dividing the medical information into a plurality of types, a plurality of pieces of secondmedical information obtained by further dividing the first medical information into a plurality of types, and third medical information associating the second medical information and degree identification information indicating a degree of the second medical information, and a user access associated information storage unit for storing user access associated information registering, in association with one another, the user identification information, and the first medical information that is accessible at least by the user identification information and the degree identification information correlated to the user identification information, where, when access to the first medical information to which the second medical information access to which is requested based on the user identification information belongs is denied based on the user access associated information, the degree identification information of the second medical information is identified based on the third medical information, and whether access is allowed or not is judged based on comparison of the degree identification information that is identified and the degree identification information of the user access associated information.

The configuration described above is effective when access to the first medical information to which the second medical information access to which is requested based on the user identification information belongs is denied based on the user access associated information

For example, a hospital clerical staff having the user identification information (for example, an ID number) who is usually in charge of medical clerical work may sometimes have to access the "second medical information" such as "drug library analysis" in the "first medical information" such as a "drug library" which is usually not related to his/her work.

At this time, with the configuration described above, for example, the work of the hospital clerical staff having the user identification information is usually not related to the "first medical information", that is, the "drug library", and access to the "drug library" is not allowed.

Therefore, the possibility of the hospital clerical staff changing the database, such as the "drug library", that is not related to his/her work by mistake may be eliminated, and the security of management of the medical information handled by the system is secured.

However, as described above, such a hospital clerical staff may also sometimes have to access a database, such as the "drug library", that is usually not related to his/her work. In this case, conventionally, a procedure of associating an authorization to exceptionally access the database with the user identification associated information of the hospital clerical staff is taken, and then access is performed, and thus the work is possibly interfered with.

Accordingly, with the configuration described above, the degree identification information of the second medical information is identifiedbasedonthe thirdmedical information, and whether access is allowed or not is judged based on comparison of the degree identification information that is identified and the degree identification information of the user access associated information.

Here, the third medical information is information associating the second medical information and the degree identification information indicating the degree of the second medical information.

That is, the "second medical information" such as "drug library analysis" and an "authorization level 6" as an example of the degree identification information are associated with each other.

Also, the degree identification information (for example, the authorization level 6) correlated to the user identification information is registered in association in the user access associated information storage unit.

Accordingly, whether the access is allowed or not is judged based on comparison of the identified degree identification information "authorization level 6" and the degree identification information (for example, authorization level 6) of the user access associated information.

For example, if the degree identification information (for example, authorization level 6) of the user access associated information is equal to or higher than the identified degree identification information, "authorization level 6", access is automatically allowed.

Accordingly, a hospital clerical staff may temporarily be allowed to access even the "second medical information" such as "analysis" of the "first medical information" in the "drug library" which he/she normally does not access, and work may be performed smoothly, and the convenience of the user is increased.

Also, this access is not unconditional, and judgement is performed based on comparison of the "degree identification information" of the "secondmedical information" access to which is desired and the "degree identification information" of the user identification information of the hospital clerical staff.

Accordingly, the convenience of the user may be increased while securing the security of the management of the medical information.

The degree identification information of the second medical information preferably includes stage degree information that is a common standard for each of the pieces of first medical information, and when the stage degree information of the degree identification information that is identified does not exceed the stage degree information of the degree identification information of the user access associated information, an access request based on the user identification information is preferably allowed under a condition of limited time.

According to the configuration described above, the degree identification information belonging to the first medical information such as the "drug library" includes stage degree information, such as an authorization level 1, an authorization level 2 or an authorization level 3, that is a common standard to each of the pieces of first medical information.

Furthermore, when the stage degree information, for example, the authorization level 6, of the degree identification information identified does not exceed the stage degree information (for example, the authorization level 6) of the degree identification information of the user access associated information, access request based on the user identification information is allowed under a condition of limited time (for example, just once).

Accordingly, even when access to the "second medical information" such as "edit drug library" that is different from the original work (work assigned in advance) of a user such as a hospital clerical staff is allowed, this is temporarily limited, and security of management of the medical information may be secured.

Preferably, there are provided the medical instrument, and a terminal device to be used by a user, and the medical information management device is connected to the medical instrument and the terminal device in a manner capable of communication.

In the present invention, the object mentioned above is achieved by a medical information management method including storing, in association with individual drug information of each drug, degree-of-effect range information obtained by dividing a degree of effect of each drug into a plurality of pieces of degree-of-effect range information, and generating administration history information of the drug used at a medical instrument for each of the pieces of degree of effect information.

### Advantageous Effects of Invention

As described above, according to the present invention, a medical information management device, a medical information management system, and a medical information management method which are capable of facilitating detailed analysis and the like of administration information of a drug may be provided.

### Brief Description of Drawings

Fig. 1 is a schematic diagram that illustrates a "drug library management system" according to an embodiment of a "medical information management system" of the present invention.
Fig. 2 (a) is a schematic diagram that illustrates a part of content of a database of a "drug library" stored in a "drug library server" in Fig. 1, and Fig. 2(b) is a schematic diagram that illustrates a part of content of a database of "hospital back office management data" as an example of a database, other than the "drug library", used in a hospital.
Fig. 3 is a schematic block diagram that illustrates a hospital clerical staff terminal illustrated in Fig. 1.
Fig. 4 is a schematic block diagram that illustrates an infusion pump illustrated in Fig. 1.
Fig. 5 is a schematic block diagram that illustrates the drug library server illustrated in Fig. 1.
Fig. 6 is a schematic block diagram that illustrates various storage units, various processing units (programs) and the like included in a first storage unit in Fig. 5.
Fig. 7 is a schematic block diagram that illustrates various storage units, various processing units (programs) and the like included in a second storage unit 30 in Fig. 5.
Fig. 8 is a schematic block diagram that illustrates various storage units, various processing units (programs) and the like included in a third storage unit 40 in Fig. 5.
Fig. 9 is a schematic flow chart that illustrates steps of acquiring, at a time of administration of a drug to a patient by an infusion pump, administration count information of the drug, alert generation information and the like.
Fig. 10 is a schematic flow chart that illustrates steps performed by a hospital clerical staff accessing the drug library server in Fig. 1 to create a "color-discriminated drug alert count graph", a "color-discriminated drug administration rate graph" and the like.
Fig. 11 is another schematic flow chart that illustrates steps performed by a hospital clerical staff accessing the drug library server in Fig. 1 to create a "color-discriminated drug alert count graph", a "color-discriminated drug administration rate graph" and the like.
Fig. 12 is another schematic flow chart that illustrates steps performed by a hospital clerical staff accessing the drug library server in Fig. 1 to create a "color-discriminated drug alert count graph", a "color-discriminated drug administration rate graph" and the like.
Fig. 13 is a schematic diagram that illustrates a drug library for each drug stored in a drug library database in Fig. 2
Fig. 14 is a schematic explanatory diagram of the color-discriminated drug alert count graph.
Fig. 15 is a schematic explanatory diagram of the color-discriminated drug administration rate graph.

### Description of Embodiments

Hereinafter, a preferred embodiment of the present invention will be described in detail with reference to the appended drawings.

Additionally, the embodiment described below is a preferred concrete example of the present invention, and is limited in various technically desirable ways, but the scope of the present invention is not limited to the mode unless the present invention is specifically limited in the following description.

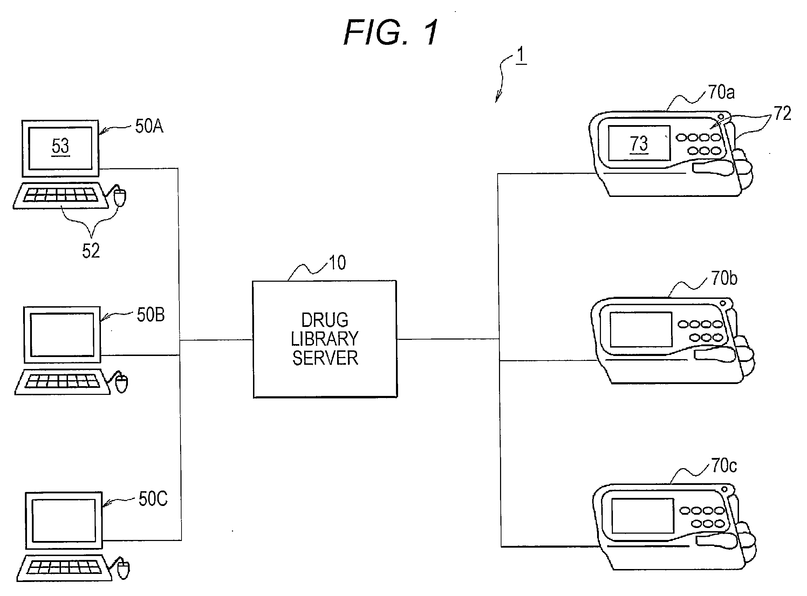

Fig. 1 is a schematic diagram that illustrates a "drug library management system 1" according to an embodiment of a "medical information management system" of the present invention.

As illustrated in Fig. 1, the drug library management system 1 includes a drug library server 10 as an example of a medical information management device. Also, hospital clerical staff terminals 50A, 50B, and 50C as examples of a terminal device are connected to the drug library server 10 in a manner capable of communication, and infusion pumps 70a, 70b, and 70c as examples of a medical instrument for drug solution injection installed at a hospital are also connected to the drug library server 10 in a manner capable of communication.

A "drug library", which is a database of data regarding prescription and the like of a drug used in the hospital, and the like are stored in the drug library server 10. This database of the "drug library" is an example of "medical information" and "first medical information".

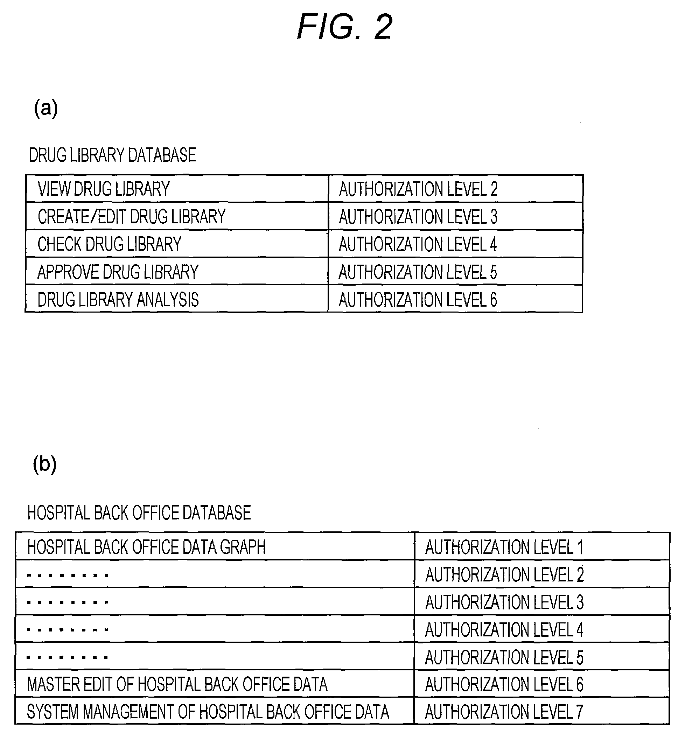

Figs. 2 (a) and 2 (b) are schematic diagrams that illustrate concrete examples of such a database.

Fig. 2 (a) is a schematic diagram that illustrates a part of content of the database of the "drug library" stored in the "drug library server 10" in Fig. 1.

The "drug library" includes detailed data regarding prescription and the like of a drug (for example, the type of the drug (anticancer drug, anesthetic), the name of the drug, the upper/lower limit values of the flow rate (mL/h), the upper/lower limit values of the injection amount (mL), prohibited administrative information, etc.), and this data is mainly transmitted to the infusion pump 70a or the like in Fig. 1, and is used at the time of administration of a drug to a patient by a medical staff such as a nurse operating the infusion pump 70a or the like.

The content illustrated in Fig. 2 (a) specifically shows the content of processing, described later, on the database of the drug library, and the authorization regarding the same.

Additionally, the infusion pump 70a illustrated in Fig. 1 is a medical instrument used at the time of accurately administering a drug to a patient, for example, and is installed at a drip infusion device or the like in the case where highly accurate control is needed at the time of administration of the drug.

Furthermore, Fig. 2(b) is a schematic diagram that illustrates a part of content of a database of "hospital back office management data", used in the hospital, as an example of a database other than the "drug library".

The "hospital back office management data" is data for managing the medical clerical work in the hospital, management data, and the like.

Accordingly, a medical staff such as a nurse, a medical clerical worker in the hospital, or a hospital clerical staff such as the manager of the hospital may smoothly use data regarding his/her work by operating the "hospital clerical staff terminal 50A" or the like in Fig. 1, and accessing the "hospital back office management data".

However, if all the hospital clerical staff, such as medical clerical workers, are allowed to freely access the database of the "drug library", the data may be changed due to erroneous operations, for example, and an unexpected situation may occur at the time of use of the "drug library".

Accordingly, an access authorization is set in the database of the "drug library" stored in the drug library server 10 in Fig. 1, and access to the database by a hospital clerical staff and the like not related to the database is restricted.

For example, the access authorization for the "drug library server 10" is granted only to the medical staff such as doctors, nurses, and pharmacists, and the access authorization is not granted to other medical clerical workers.

Additionally, in Fig. 2 (a), five levels of authorization levels 2 to 6 are illustrated, but this is not restrictive.

On the other hand, the access authorization for the database of the "hospital back office management data" illustrated in Fig. 2 (b) (not illustrated in Fig. 1) is granted only to the medical clerical workers and administrative staff, and the access authorization is not granted to the medical staff such as doctors.

Also, in the case of accessing the "drug library" or "hospital back office management data", a plurality of processing contents are set. For example, in the case of the "drug library", processes such as viewing, composing/editing, checking and approval, drug library analysis and the like illustrated in Fig. 2 (a) are set, and in the case of the "hospital backoff ice management data", processes regarding a graph, master editing, system management and the like illustrated in Fig. 2 (b) are set. These processing contents are examples of "second medical information".

Moreover, an "authorization level" as an example of "degree identification information", depending on whose degree execution is allowed, is set for the processing content. Only the hospital clerical staff having such an "authorization level" is enabled to execute the process. Specifically, as described later, the authorization level is linked with an ID number as an example of "user identification information" of a hospital clerical staff.

Additionally, in Fig. 2(b), seven levels of authorization levels 1 to 7 are illustrated, but this is not restrictive.

Now, the drug library server 10, the hospital clerical staff terminals 50A and the like, and the infusion pumps 70a and the like illustrated in Fig. 1 each include a computer, and the computer includes a CPU (Central Processing Unit), a RAM (Random Access Memory), a ROM (Read Only Memory) and the like, not illustrated, and these are connected by a bus.

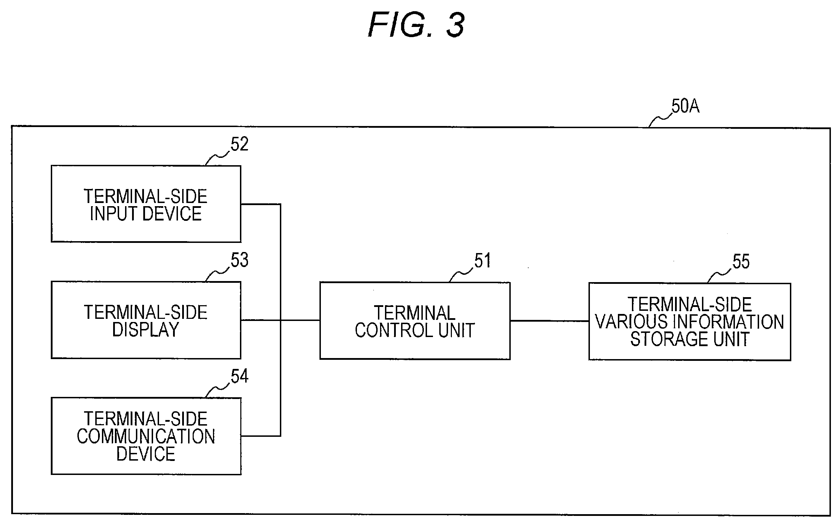

Fig. 3 is a schematic block diagram that illustrates the hospital clerical staff terminal 50A illustrated in Fig. 1.

As illustrated in Fig. 3, the hospital clerical staff terminal 50A includes a terminal control unit 51. The terminal control unit 51 is configured to control a "terminal-side input device 52" as an example of an input device for inputting data and the like, a "terminal-side display 53" as an example of a display unit for displaying data and the like, and a "terminal-side communication device 54" for performing communication with the drug library server 10 and the like, and to also control a "terminal-side various information storage unit 55".

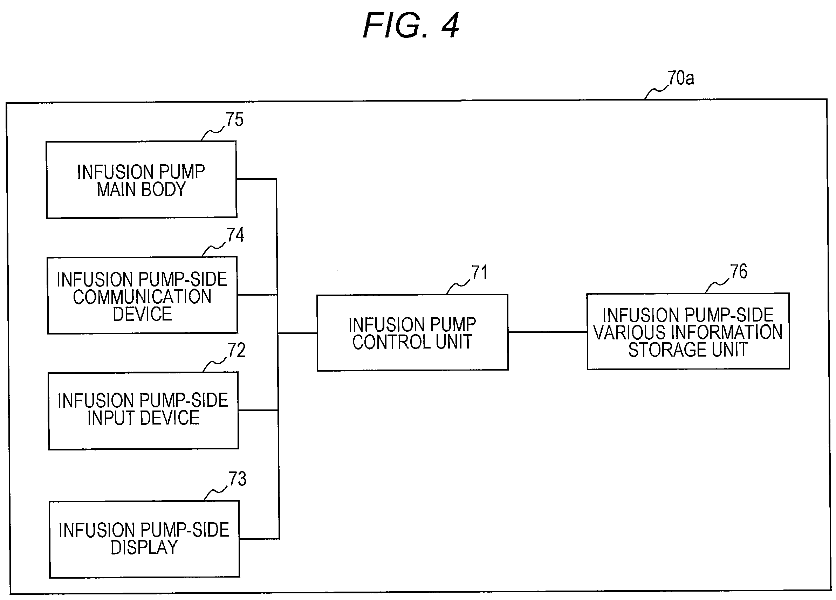

Fig. 4 is a schematic block diagram that illustrates the infusion pump 70a illustrated in Fig. 1.

As illustrated in Fig. 4, the infusion pump 70a includes an infusion pump control unit 71. The infusion pump control unit 71 is configured to control an "infusion pump-side input device 72" as an example of an input device, such as a dial or input buttons, for inputting data and the like, an "infusion pump-side display 73" as an example of a display unit for displaying data and the like, an "infusion pump-side communication device 74" for performing communication with the drug library server 10 and the like, and an "infusion pump main body 75" for performing pump operation of the infusion pump 70a, and to also control an "infusion pump-side various information storage unit 76".

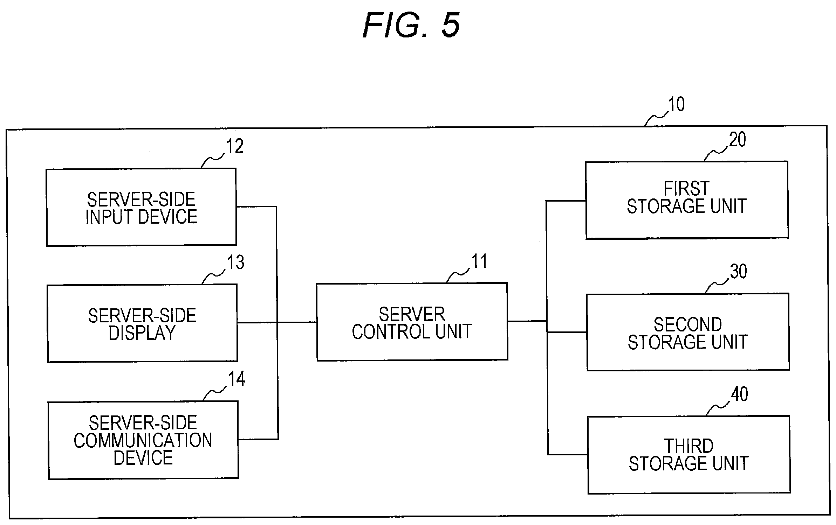

Fig. 5 is a schematic block diagram that illustrates the drug library server 10 illustrated in Fig. 1.

As illustrated in Fig. 5, the drug library server 10 includes a "server control unit 11". The server control unit 11 is configured to control a "server-side input device 12" for inputting data and the like, a "server-side display 13" for displaying data and the like, and a "server-side communication device 14" for performing communication with the infusion pump 70a or the like, and the hospital clerical staff terminal 50A or the like.

Also, as illustrated in Fig. 5, the server control unit 11 is configured to control a "first storage unit 20", a "second storage unit 30", a "third storage unit 40", and the like.

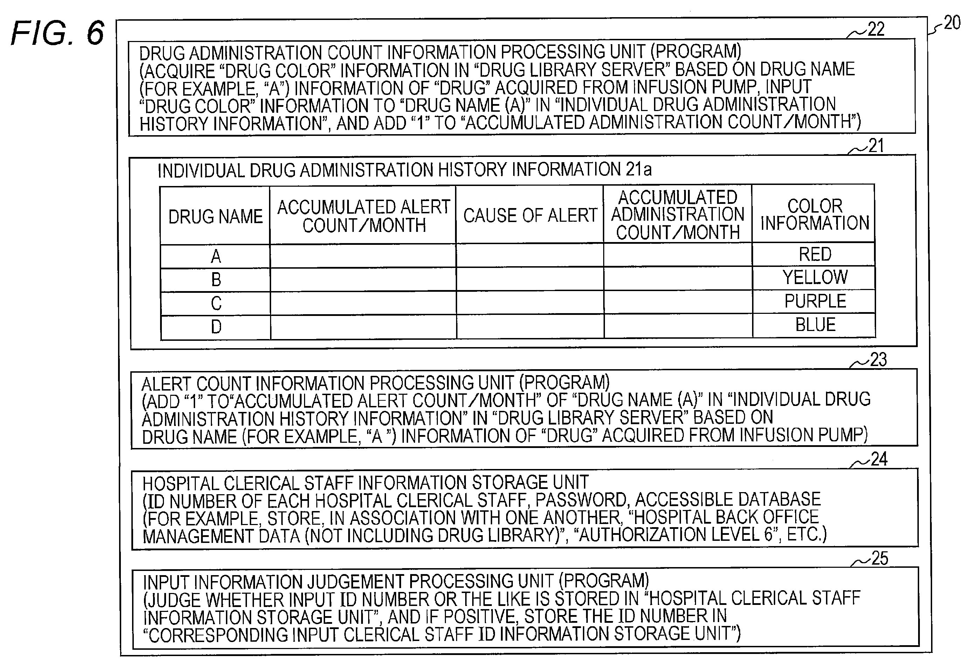

Figs. 6 to 8 are each a schematic block diagram that illustrates various storage units, various processing units (programs) and the like stored in the first storage unit 20, the second storage unit 30, or the third storage unit 40 in Fig. 5. Specifics regarding these will be given later.

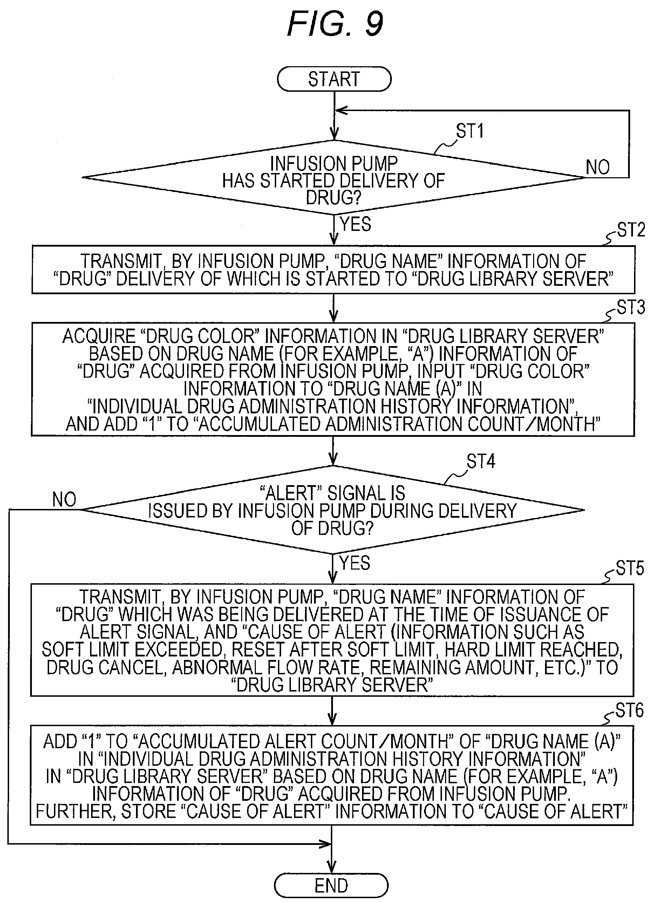

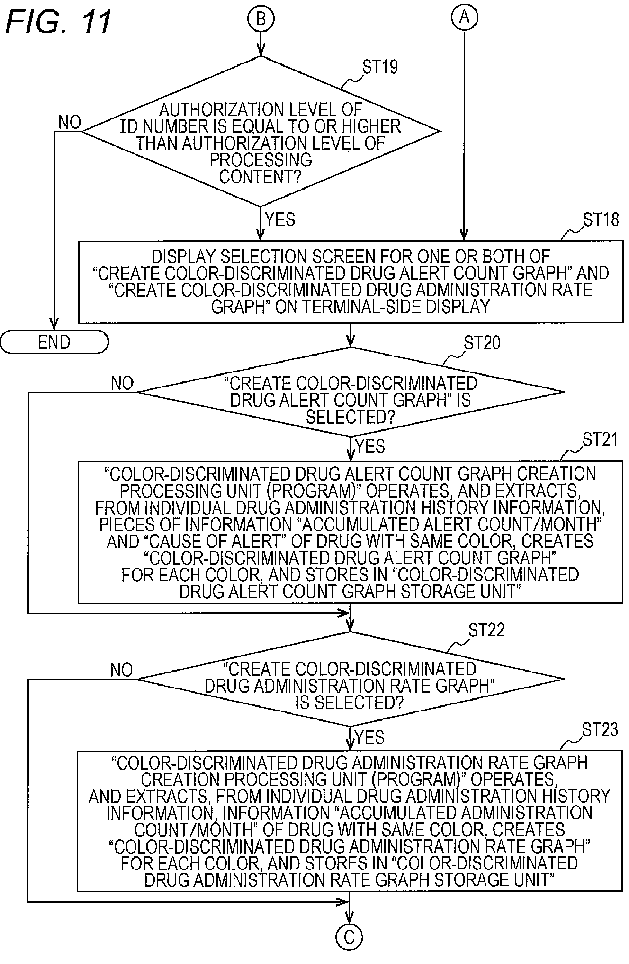

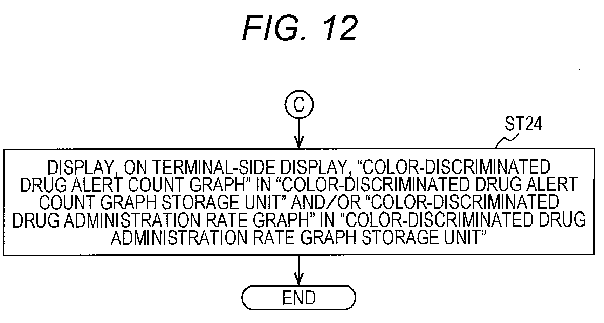

Figs. 9 to 12 are each a schematic flow chart that illustrates an example operation of the drug library management system 1 according to the embodiment of the present invention. In the following, the operations will be described with reference to the flow charts in Figs. 9 to 12, and configurations in Figs. 1 to 8 will also be described.

Fig. 9 is a schematic flow chart that illustrates a step of acquiring, at a time of administration a drug to a patient by the infusion pump 70a or the like, administration count information (an example of administration record information) of the drug, alert (an example of warning information) generation information and the like.

As cause information for generation of the alert, there may be cited information such as "soft limit exceeded" and "reset after soft limit", which are pieces of drug administration limitation information, "hard limit reached" and "drug cancel", which are pieces of absolute administration limitation information stricter than the soft limit, and pieces of information such as "abnormal flow rate" of drug administration, the "remaining amount" of a drug solution, and the like.

First, in ST1 in Fig. 9, whether the "infusion pump main body 75" of the infusion pump 70a in Fig. 4 operated, and whether the infusion pump 70a has started delivery of a drug is judged.

If the infusion pump 70a is judged in ST1 to have started delivery of a drug, the process proceeds to ST2. In ST2, the infusion pump 70a transmits "drug name" information, such as "drug A", of the "drug" delivery of which was started, to the "drug library server 10". Specifically, transmission is performed via the "infusion pump-side communication device 74" in Fig. 4.

Next, the process proceeds to ST3. In ST3, based on the drug name (for example, "A") information of the "drug" acquired by the "drug library server 10" from the infusion pump 70a, a "drug color" associated with the drug A, such as information of red, is obtained from the drug library for the drug A in the "drug library database" in the "drug library server 10".

The "drug color" is an example of "degree-of-effect range information" and "color information". Also, the "drug library for the drug A" is an example of "individual drug information".

Here, the "drug color" will be described.

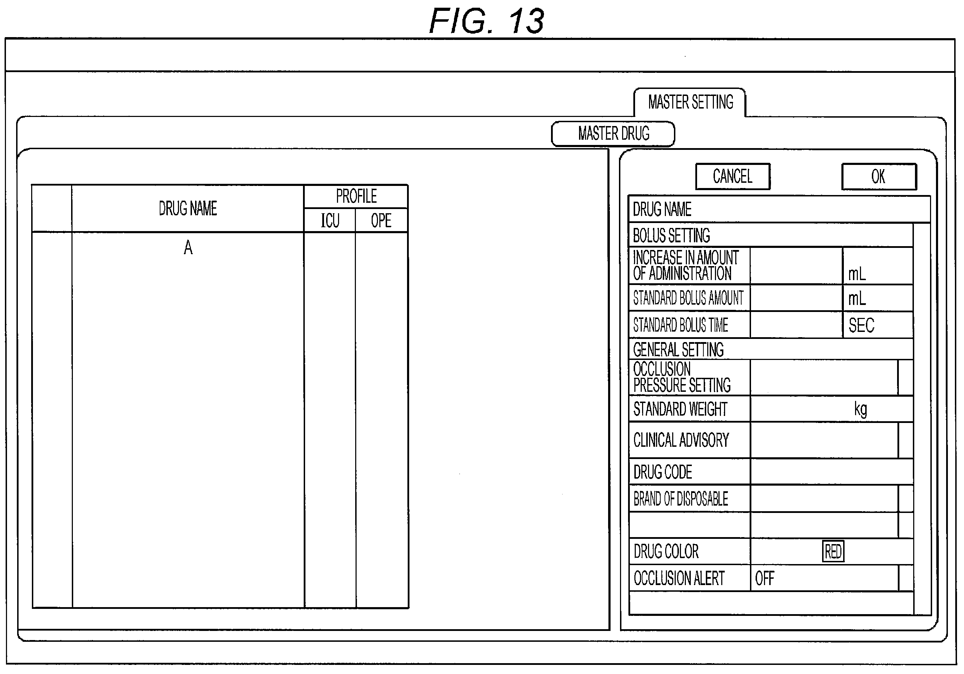

Fig. 13 is a schematic diagram that illustrates the drug library of an individual drug stored in the drug library database in Fig. 2.

As illustrated in Fig. 13, other than the drug name, pieces of information about the "amount of administration", "occlusion pressure" and the like are recorded in the drug library for a drug (for example, the drug name "A"), and in the present embodiment, information about the "drug color" is also recorded.

This information about the drug color indicates a color according to the degree of risk at the time of administration of the drug (the degree of attention, as an example of the degree of effect, required for administration due to a strong effect or efficacy).

That is, drugs are sorted into groups in the descending order of the degree of risk of damage or second effect from medicines, and each group is associated with the color information.

For example, "red", "purple red", "yellow green","yellow", blue green", "purple", "green", "blue", "reddish brown", "olive", and "navy" are assigned in order from the group with the highest degree of risk.

Accordingly, a user may swiftly discriminate whether the degree of risk of a drug is high or not by viewing the color displayed on a display or the like.

Now, returning to ST3, the "drug library server 10" acquires information of the drug color "red" (see Fig. 13) from the drug library for the drug A, and also inputs, in "drug name (A) " in "individual drug administration history information 21a" in an "individual drug administration history information storage unit 21" in Fig. 6, the "drug name information" of the drug A and the information "drug color (red) ", and adds "1" to "accumulated administration count/month". Additionally, when the pieces of information, the "drug name (A)" and the "drug color (red)", are already input, only the information about the "accumulated administration count/month" is input.

Moreover, these steps are performed by operation of a "drug administration count information processing unit (program) 22" in Fig. 6.

Also, the "individual drug administration history information 21a" is an example of "administration history information".

Next, the process proceeds to ST4. In ST4, whether the infusion pump 70a has issued an "alert" signal during delivery of the drug A is judged.

In ST4, if the infusion pump 70a has generated an "alert" signal, the infusion pump 70a transmits, to the "drug library server 10", the "drug name" information of the drug which was being delivered at the time of issuance of the alert signal, such as the drug A, and a "cause of alert (information such as soft limit exceeded, reset after soft limit, hard limit reached, drug cancel, abnormal flow rate, remaining amount, etc.)".

Then, the process proceeds to ST6. In ST6, based on the drug name (for example, "A") information of the "drug" acquired from the infusion pump 70a, "1" is added to "accumulated alert count/month" of the "drug name (A)" in the "individual drug administration history information 21a" in Fig. 6 in the "drug library server 10". Also, the "cause of alert" information is input to the section of the "cause of alert".

These steps are performed by an "alert count information processing unit (program) 23" in Fig. 6.

The "accumulated alert count/month" and the "cause of alert", and the "accumulated administration count/month" are thus stored in the "individual drug administration history information 21a" in Fig. 6 in association with the drug name A, and the "color information" indicating the degree of risk of the drug is also stored in association.

Steps of acquiring the administration count information of a drug, the alert generation information and the like at the time of administration of the drug to a patient by the infusion pump 70a or the like are thus ended.

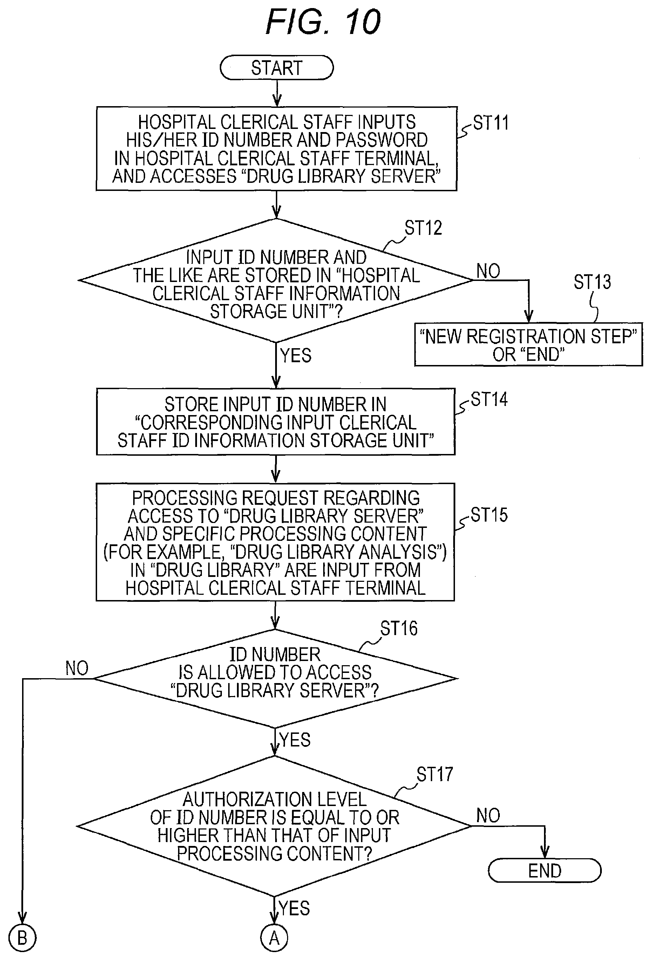

Figs. 10 to 12 are schematic flow charts that illustrate steps performed by a hospital clerical staff accessing the drug library server 10 in Fig. 1 to create a "color-discriminated drug alert count graph", a "color-discriminated drug administration rate graph" and the like.

First, hospital clerical staff as an example of users trying to access the drug library server 10 in Fig. 1 each have an ID number or the like as user identification information.

Accordingly, as indicated by ST11, a hospital clerical staff trying to access the drug library server 10 in Fig. 1 inputs, by using the hospital clerical staff terminal 50A illustrated in Fig. 1, his/her ID number, a password and the like in his/her hospital clerical staff terminal 50A.

Then, in ST12, whether the input ID number and the like are stored in "hospital clerical staff information storage unit 24" in Fig. 6 is judged. As illustrated in Fig. 6, the ID number of a hospital clerical staff and a "password" correlated to the ID number are registered in the "hospital clerical staff information storage unit 24".

Accordingly, whether the inputting person is already registered may be judged by judgement of storage/non-storage in the "hospital clerical staff information storage unit 24", and access to the "drug library server 10" by a non-registered person may be prevented.

Specifically, this operation is performed by an "input information judgement processing unit (program) 25" in Fig. 6.

If it is judged in ST12 that the input ID number and the like are not registered in the "hospital clerical staff information storage unit 24", a "new registration step" is performed and new registration is made, or the present step is ended with no new registration being made, as indicated by ST13.

If it is judged in ST12 that the input ID number and the like are registered in the "hospital clerical staff information storage unit 24", the process proceeds to ST14.

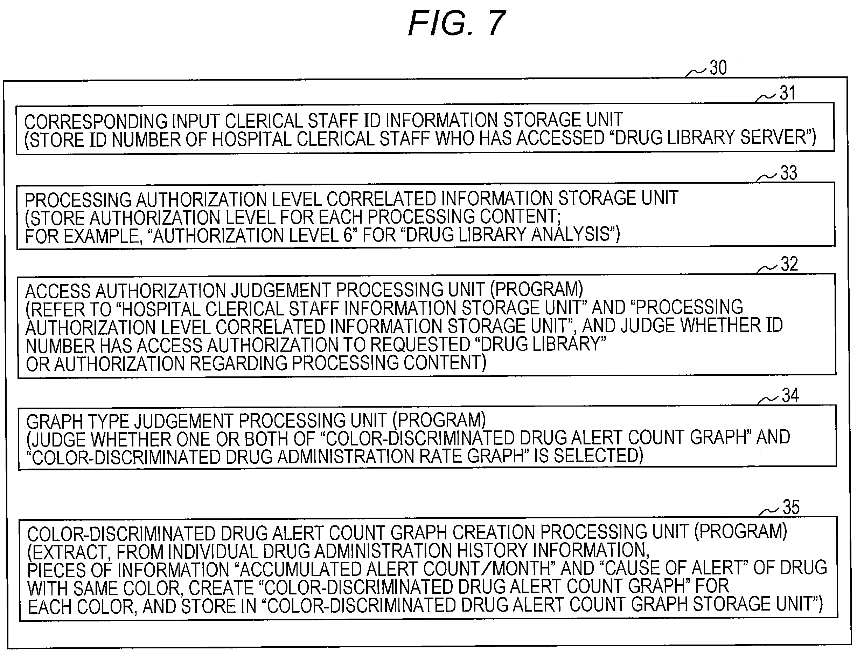

In ST14, the input ID number is stored in a "corresponding input clerical staff ID information storage unit 31" in Fig. 7.

Next, the process proceeds to ST15. In ST15, the hospital clerical staff inputs, from his/her hospital clerical staff terminal 50A, the "drug library database" which is the database that he/she wants to access and the "processing content".

For example, "drug library analysis (for example, creation of a "color-discriminated drug alert count graph", "color-discriminated drug administration rate graph" and the like) is input as the processing content (see Fig. 2(a)).

Then, the process proceeds to ST16. In ST16, whether the ID number is associated with the access authorization for the drug library database is judged.

Specifically, an "access authorization judgement processing unit (program) 32" in Fig. 7 operates, and refers to the "corresponding input clerical staff ID information storage unit 31" in Fig. 7 and the "hospital clerical staff information storage unit 24" in Fig. 6, and judges whether the "drug library database" is included in "accessible database" of this ID number.

If it is judged in ST16 that this ID number is allowed to access the "drug library database", the process proceeds to ST17.

On the other hand, a case where there is no coincidence in ST16 will be described later.

In ST17, whether the registered authorization level of the ID number, that is, the "authorization level" registered in the "hospital clerical staff information storage unit 24" in Fig. 6 in association with the ID number, is equal to or higher than the "authorization level" of the " drug library analysis", which is the input processing content, is judged.

Specifically, judgement is made by the "access authorization judgement processing unit (program) 32". That is, judgement is made by referring to a "processing authorization level correlated information storage unit 33" in Fig. 7, and this "processing authorization level correlated information storage unit 33" stores an "authorization level 6" as an example of stage degree information of the "authorization level" of each processing content such as "drug library analysis (for example, creation of the "color-discriminated drug alert count graph", the "color-discriminated drug administration rate graph" and the like)" in Fig. 2(a).

Accordingly, after acquiring the "authorization level" that is registered in the "hospital clerical staff information storage unit 24" in association with the ID number, the "access authorization judgement processing unit (program) 32" judges whether this "authorization level" is equal to or higher than the "authorization level 6" of the processing content, the "drug library analysis", stored in the "processing authorization level correlated information storage unit 33".

Accordingly, a "hospital clerical staff information storage unit 24" is an example of a "user access associated information storage unit", and the "processing authorization level correlated information storage unit 33" is an example of a "third medical information storage unit".

If, in ST17, the "authorization level" associated with the ID number is not equal to or higher than the "authorization level 6" of "drug library analysis" as an example of input processing content, the hospital clerical staff who input the ID number is denied access as not having the authorization to perform the processing content.

On the other hand, if, in ST17, the "authorization level" associated with the ID number is equal to or higher than the "authorization level 6" of "drug library analysis" as an example of input processing content, the process proceeds to ST18.

In ST18, the hospital clerical staff who input the ID number is assumed to have the authorization to perform "drug library analysis", which is the processing content, and "drug library analysis", which is the processing content, is performed.

Specifically, a selection screen for selecting one or both of "create color-discriminated drug alert count graph" and "create color-discriminated drug administration rate graph" is displayed on the "terminal-side display 53".

That is, the "color-discriminated drug alert count graph" is a graph that displays, for each drug color, the count information of alerts generated at the time of use of drugs with the same "drug color" information described above. Accordingly, the number of times of alert generation for each drug color may be easily analyzed.

On the other hand, the "color-discriminated drug administration rate graph" is a graph that displays, for each drug color, the administration rate of drugs with the same "drug color" information described above. Accordingly, the administration rate of each drug color may be easily analyzed.

As described above, in the present embodiment, if a hospital clerical staff is in charge of medical clerical work, and is not a medical staff such as a doctor, a nurse, or a pharmacist, he/she is basically denied the access authorization regarding the "drug library database", which is a database regarding prescription and the like of a drug, and the security of management of the data is secured.

On the other hand, such a person in charge of the medical clerical work is allowed to access the "hospital back office management data", which is the database in Fig. 2(b), and the convenience is secured.

However, a person in charge of the medical clerical work may also be in charge of management and be a doctor, and such a person may, in the course of his/her work, have to access a database that is not directly related to his/her original work, such as the "drug library database".

If access to the database is uniformly denied in such a case, and registration of the access authorization has to be made each time, work would be interfered with, and thus, in the present embodiment, the following process is performed.

First, in ST16, if the ID number has the access authorization only for the "hospital backoff ice management data", and the access authorization for the drug library database is not registered in the "hospital clerical staff information storage unit 24", this person is denied access to the "drug library database" even when requesting access with his/her ID number.

Next, the process proceeds to ST19 in Fig. 9. In ST19, information about the registered "authorization level" of the ID number, that is, the "authorization level" stored in the "hospital clerical staff information storage unit 24" such as the "authorization level 6", is acquired, and is compared with the "authorization level 6" of "drug library analysis" which is the processing content. Then, whether the registered authorization level of the ID number is equal to or higher than the "authorization level 6" of "drug library analysis" is judged.

Specifically, judgement is performed by the "access authorization judgement processing unit (program) 32".

If it is judged in ST19 that the registered authorization level of the ID number is not equal to or higher than the "authorization level 6" of "drug library analysis", it is judged that there is no need to allow an exceptional access authorization for a database for which the access authorization is not granted in advance, and the access request for "approve" in the drug library database is denied.

In this case, in order for the hospital clerical staff with the ID number to access the "drug library database" and perform the process of "drug library analysis", he/she has to make an application to the administrator or the like, and the memory in the "hospital clerical staff information storage unit 24" in Fig. 6 has to be changed. Exceptional accesses, by hospital clerical staff, that are not much needed may thereby be limited, and the security regarding management of medical information may be secured.

On the other hand, if it is judged in ST19 that the registered authorization level of the ID number is equal to or higher than the "authorization level 6" of "drug library analysis", a need to allow an exceptional access to the hospital clerical staff is recognized, and the process proceeds to ST18.

In ST18, the hospital clerical staff who input the ID number is assumed to have the authorization to perform "drug library analysis", which is the processing content, and "drug library analysis", which is the processing content, is performed.

Specifically, the selection screen for selecting one or both of "create color-discriminated drug alert count graph" and "create color-discriminated drug administration rate graph" is displayed on the terminal-side display 53.

However, each permission of the execution goes through the permission judgement step, and permission has to be given each time. This "permission for one time" is an example of "limited time".

As described above, in the present embodiment, even if an access authorization for the drug library database is not registered in advance in the "hospital clerical staff information storage unit 24", access may be allowed as an exception, and interference with work may be prevented.

Also, when allowing access as an exception, as in the case described above, access is allowed only when the numerical value which is the stage degree information regarding the authorization level of processing content is equal or greater, and thus the security of management of medical information may be secured, and also the convenience of the user may be increased.

Next, the process proceeds to ST20. In ST20, a "graph type judgement processing unit (program) 34" in Fig. 7 operates, and whether "create color-discriminated drug alert count graph" is selected is judged.

If "create color-discriminated drug alert count graph" is selected in ST20, the process proceeds to ST21. In ST21, a "color-discriminated drug alert count graph creation processing unit (program) 35" in Fig. 7 operates, and extracts, from the "individual drug administration history information 21a" in Fig. 6, pieces of information "accumulated alert count/month" and "cause of alert" of a drug with the same color as the drug A, for example.

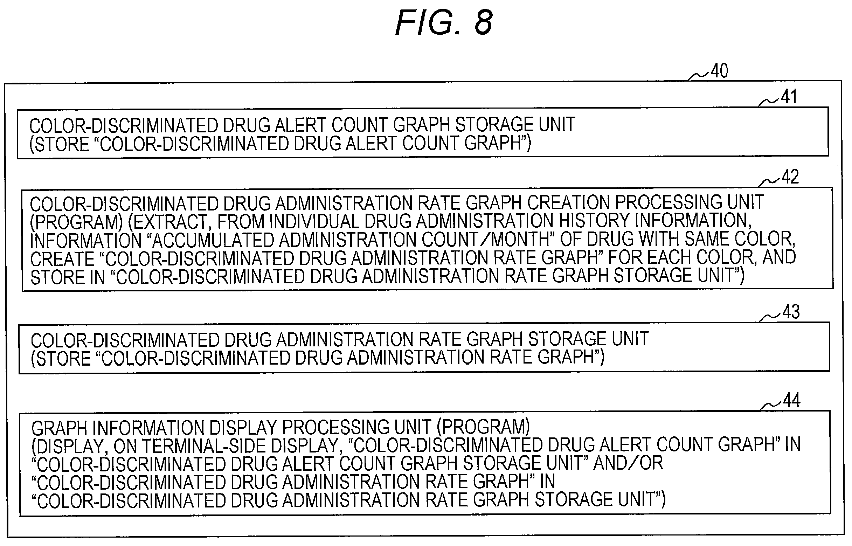

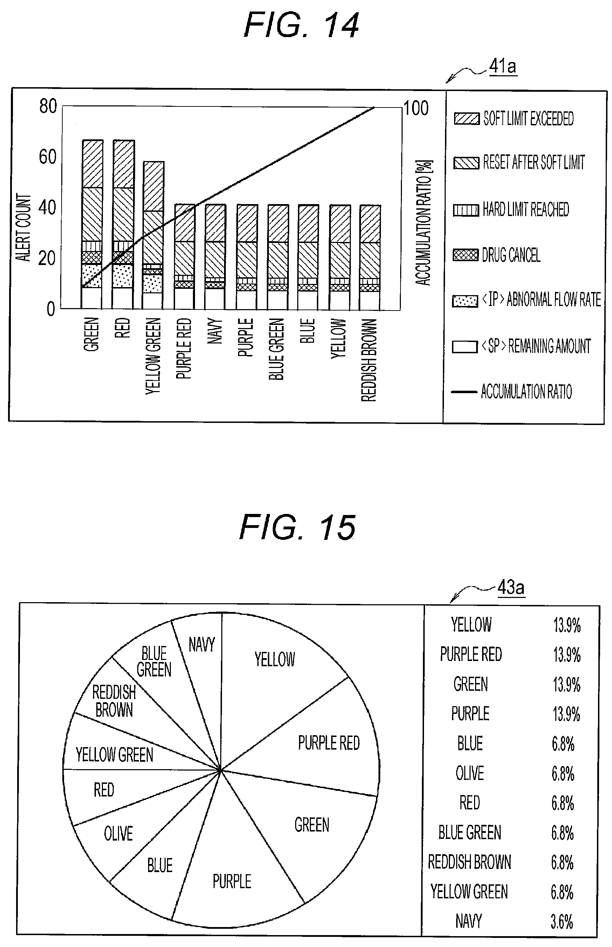

Then, a "color-discriminated drug alert count graph 41a" as illustrated in Fig. 14 is created, and is stored in a "color-discriminated drug alert count graph storage unit 41" in Fig. 8.

Fig. 14 is a schematic explanatory diagram of the color-discriminated drug alert count graph 41a.

As illustrated in Fig. 14, the "color-discriminated drug alert count graph 41a" indicates, for each group of drugs associated with the same color information, such as red, the number of times of alert generation at the infusion pump 70a or the like on a per-month basis.

Moreover, this graph is created in such a way as to allow grasping of the number of times of generation for each cause of alert generation (soft limit exceeded, etc.).

Accordingly, a user viewing such a graph may swiftly grasp the degree of risk of a drug by the color information, and also may easily grasp the frequency and the cause of alert generation of a drug in the group of the degree of risk indicated by the same color, and as a result, analysis is facilitated.

Next, the process proceeds to ST22. In ST22, the "graph type judgement processing unit (program) 34" in Fig. 7 operates, and whether "create color-discriminated drug administration rate graph" is selected is judged.

If "create color-discriminated drug administration rate graph" is selected in ST22, the process proceeds to ST23. In ST23, a "color-discriminated drug administration rate graph creation processing unit (program) 42" in Fig. 8 operates, and extracts, from the "individual drug administration history information 21a" in Fig. 6, information "accumulated administration count/month" of the drug with the same color.

Then, a "color-discriminated drug administration rate graph 43a" as illustrated in Fig. 15 is created, and is stored in a "color-discriminated drug administration rate graph storage unit 43" in Fig. 8.

Fig. 15 is a schematic explanatory diagram of the color-discriminated drug administration rate graph 43a.

As illustrated in Fig. 15, the "color-discriminated drug administration rate graph 43a" indicates, for each group of drugs associated with the same color information, such as red, the administration rate (%) at the infusion pump 70a or the like on a per-month basis.

Accordingly, a user viewing such a graph may swiftly grasp the degree of risk of a drug by the color information, and also may easily grasp the administration rate (%) of a drug in the group of the degree of risk indicated by the same color, and as a result, analysis is facilitated.

Next, the process proceeds to ST24. In ST24, a "graph information display processing unit (program) 44" in Fig. 8 operates, and displays, on the "terminal-side display 53" of the user, the "color-discriminated drug alert count graph 41a", illustrated in Fig. 14, in the "color-discriminated drug alert count graph storage unit 41" and/or the "color-discriminated drug administration rate graph 43a", illustrated in Fig. 15, in the "color-discriminated drug administration rate graph storage unit 43".

Accordingly, as described above, a user viewing these graphs may easily grasp the number of times of alert, the administration rate and the like according to the degree of risk of the drug, and also detailed analysis of administration information of the drug is enabled.

Incidentally, the present invention is not limited to the embodiment described above. The present embodiment gives description citing the infusion pump 70a or the like as an example of a medical instrument for drug solution injection, but the present invention is not limited to such, and may be suitably applied to other medical instruments such as a syringe pump.

### Reference Signs List

1 Drug library management system
10 Drug library server
11 Server control unit
12 Server-side input device
13 Server-side display
14 Server-side communication device
20 First storage unit
21 Individual drug administration history information storage unit
21a Individual drug administration history information
22 Drug administration count information processing unit (program)
23 Alert count information processing unit (program)
24 Hospital clerical staff information storage unit
25 Input information judgement processing unit (program)
31 Corresponding input clerical staff ID information storage unit
32 Access authorization judgement processing unit (program)
33 Processing authorization level correlated information storage unit
34 Graph type judgement processing unit (program)
35 Color-discriminated drug alert count graph creation processing unit (program)
41 Color-discriminated drug alert count graph storage unit
41a Color-discriminated drug alert count graph
42 Color-discriminated drug administration rate graph creation processing unit (program)
43 Color-discriminated drug administration rate graph storage unit
44 Graph information display processing unit (program)
50A Hospital clerical staff terminal
51 Terminal control unit
52 Terminal-side input device
53 Terminal-side display
54 Terminal-side communication device
55 Terminal-side various information storage unit
70a Infusion pump
71 Infusion pump control unit
72 Infusion pump-side input device
73 Infusion pump-side display
74 Infusion pump-side communication device
75 Infusion pump main body
76 Infusion pump-side various information storage unit

## Claims

1. A medical information management device,
wherein the medical information management device stores, in association with individual drug information of each drug, degree-of-effect range information obtained by dividing a degree of effect of each drug into a plurality of pieces of degree-of-effect range information, and
the medical information management device generates administration history information of the drug used at a medical instrument for each of the pieces of degree of effect information.

2. The medical information management device according to claim 1,
wherein the degree-of-effect range information is color information, and
the administration history information includes warning information generated at a time of use of the drug at the medical instrument, and administration record information of the drug, and the warning information and the administration record information are displayed on a display unit together with the color information.

3. The medical information management device according to claim 2, wherein the warning information is generated in association with cause information regarding the warning information.

4. The medical information management device according to any one of claims 1 to 3, comprising:
a third medical information storage unit for storing
user identification information for identifying a user who uses medical information,
a plurality of pieces of first medical information obtained by dividing the medical information into a plurality of types,
a plurality of pieces of second medical information obtained by further dividing the first medical information into a plurality of types, and
third medical information associating the second medical information and degree identification information indicating a degree of the second medical information; and
a user access associated information storage unit for storing user access associated information registering, in association with one another, the user identification information, and the firstmedical information that is accessible at least by the user identification information and the degree identification information correlated to the user identification information,
wherein, when access to the first medical information to which the secondmedical information access to which is requested based on the user identification information belongs is denied based on the user access associated information, the degree identification information of the second medical information is identified based on the third medical information, and whether access is allowed or not is judged based on comparison of the degree identification information that is identified and the degree identification information of the user access associated information.

5. The medical information management device according to claim 4,
wherein the degree identification information of the second medical information includes stage degree information that is a common standard for each of the pieces of first medical information, and
when the stage degree information of the degree identification information that is identified does not exceed the stage degree information of the degree identification information of the user access associated information, an access request based on the user identification information is allowed under a condition of limited time.

6. A medical information management system comprising:
the medical instrument;
a terminal device to be used by a user; and
a medical information management device according to any one of claims 1 to 5, the medical information management device being connected to the medical instrument and the terminal device in a manner capable of communication.

7. A medical information management method comprising:
storing, in association with individual drug information of each drug, degree-of-effect range information obtained by dividing a degree of effect of each drug into a plurality of pieces of degree-of-effect range information; and
generating administration history information of the drug used at a medical instrument for each of the pieces of degree of effect information.
